# EUROPEAN PATENT SPECIFICATION

(11) **EP 3 515 370 B1**
(45) Date of publication and mention of the grant of the patent: **28.06.2023**
(21) Application number: 17852557.2
(22) Date of filing: 19.09.2017
(51) Int. Cl.: A61F 2/46, A61B 17/56, A61B 17/92

(54) **AN AUTOMATED SLAPHAMMER TO REMOVE ORTHOPAEDIC IMPLANTS**
AUTOMATISIERTER AUSSCHLÄGER ZUR ENTFERNUNG VON ORTHOPÄDISCHEN IMPLANTATEN
MARTEAU À IMPACT AUTOMATIQUE POUR RETIRER DES IMPLANTS ORTHOPÉDIQUES

(30) Priority: 20.09.2016 IN 201621031987
(43) Date of publication of application: 31.07.2019
(73) Proprietor: Chhatrala, Pankajkumar K., Gujarat Halvad 363330 (IN)
(72) Inventor: Chhatrala, Pankajkumar K., Gujarat Halvad 363330 (IN)
(74) Representative: ABG Intellectual Property Law, S.L.
(86) International application number: PCT/IN2017/050413
(87) International publication number: WO 2018/055639

(56) References cited:
- EP-A1- 1 834 737
- WO-A1-2016/112397
- FR-A1- 2 868 977
- FR-B1- 2 868 977
- US-A- 4 919 216
- US-A1- 2012 215 267

## Description

### FIELD OF THE INVENTION:

The present invention relates to tools for facilitating orthopaedic procedure. The present invention relates generally to implant and systems for orthopaedic applications. More specifically, the present invention relates to implant extraction with coupling gear for attachment to manual and power force transducers with control over force variables. More particularly, this invention relates to the device that can be used by a surgeon to remove articles embedded in bone, such as a prosthetic, nails or implants.

### BACKGROUND OF THE INVENTION:

Injuries, disease, as well as the natural aging process, can lead to changes in the musculoskeletal system of the body i.e. the bones, joints, and muscles of the body. Such changes, or injuries, can manifest in physical degeneration. To correct such damage, medical practitioners routinely perform various orthopaedic procedures.

Routine orthopaedic procedures include hip replacement and knee replacement surgery. During replacement of a joint such as the hip or knee, diseased or damaged joint surfaces are replaced with metal and plastic components shaped to allow continued use (and motion) of the joint. After incisions are made for surgical access, damaged or diseased materials, such as bones and muscles, are removed, with artificial prostheses inserted in their place. Additionally, it is often necessary to remove surgical implements that are secured to a patient. However, access to the surgical implements may be limited due to the confines of the surgical operating workplace. Apart from joints replacement, intramedullary nails (IMN) are used for internal fixation of fractured long bones. IMN is required to remove frequently because of various reasons i.e. infection, non-union of fractures, secondary fracture with IMN in place, continuous pain, psychological reasons etc.

Several different tools are used during orthopaedic procedures to place and/or remove various objects. Mallets are frequently used to apply an impacting force on a medical tool, such as a chisel, to remove bones or other implanted objects. Mallets are also commonly used to insert an implant, and to remove tools positioned in the surgical area. While mallets are effective, the impacting force must be axially applied to avoid misalignment of the prosthesis, or the inadvertent removal of bone. Moreover, the force applied must be sufficiently controlled for avoiding damage to the bone.

Slap hammers are commonly used as extraction tools for withdrawal or removal of stuck implants/objects form bone during orthopaedic surgery. This may be achieved typically by first engaging the proximal stem with a distal end of a slap hammer assembly and urging a moveable slap weight proximally against a stationary handle or slap weight. This is intended to break the bond at the stem and bone interface and eventually withdraw the prosthesis by application of sufficient axial load. Engagement between the known slap hammer extraction tool and stem is preferably via co-operating threads, the event of a screwed stem; an extraction tool may engage via a hex nut and withdraw by opposite rotation of the stem.

Many orthopaedic procedures involve implants for replacing damaged and dysfunctional joints. For example, revision surgeries of total joint replacement (TJR) and hemi arthroplasty (replacing one-half of the joint) procedures have been developed. Hips, knees, elbows, shoulders and wrists are commonly reconstructed with implants, such as prosthetic joints that are designed for optimal wear, comfort, biocompatibility and performance. Such replacement joint implants have benefited many patients by restoring their mobility and other functions.

Stem/implant which was implanted during previous orthopaedic surgery to support bone or reconstruct normal skeletal structure (i.e. broken bone, damaged joints etc.). Over the period of time bone tissue in grows into implants in-order to supports healing bone. Because of ingrown bone tissue it is difficult to remove implant from bone.

Intra medullary Nail (IMN) is inserted to stabilize fractured long bones i.e femur, tibia. Over the period of time bone heals over IMN and bone tissue grows over IMN surface, resulting bio-bonding between bone tissue and implant surface As a result whenever it is required to remove IMN, Surgeon has to put serious effort to remove IMN from Bone. Currently Orthopaedic surgeon fix intramedullary nail with malleus like instrument and removes IMN by reverse stroking using specially designed hammer.

Orthopaedic revision procedures are necessitated in case of prosthetic/implant failures from various causes. For example, further deterioration and trauma can lead to prosthetic joint failures. Another problem relates to loosening and disengagement of the components. For example, orthopaedic cement, which is commonly used to bond prosthetic components to bone, can loosen and disengage. Looseness and "play" in implants, such as prosthetic joints, can cause significant problems. These include patient discomfort and immobility. Moreover, such looseness can increase under dynamic loading, and can ultimately lead to complications associated with implant failure.

When revision procedures are indicated by such conditions, extracting existing implants and the cement mantels bonding same can present significant difficulties. Extracting prostheses that have been permanently bonded in place with high-strength adhesives can require substantial force, with resulting trauma and collateral damage. For example, perforated and cracked existing bone structures can result from forces associated with extracting failed prostheses. Moreover, implants can become stuck during installation. For example, if the cavity formed for the implant shaft is too small, a test fit can result in immobility with resistance to both insertion and extraction. Extracting a stuck implant can require breaking the surrounding bone structure, with resulting complications.

The prior art has attempted to address some of the problems associated with orthopaedic implant extractions. For example, the Engelbrecht et al. U.S. Pat. No. 4,248,232 discloses the use of a vibrating tool to soften the cement between nested components bonded together. The Hood et al. U.S. Pat. No. 5,045,054 discloses an ultrasound power generator adapted for coupling to end prostheses and vibrating same to soften their adhesive bonds. Hood et al. disclose an ultrasonic tool for attachment to and removal of surgical components in U.S. Pat. No. 5,318,570. Vandewalle et al. U.S. Pat. No. 6,190,392 disclose an auger tool connected to an ultrasonic transducer/hand piece for extracting an osteal cement mantel. US Pat. No. 5,913,860 discloses the device for impaction and extraction of nail from the bone. This device has slap and hammer which can be removed or attached to slide rod used for removal of the nail.

Several different tools have been developed to facilitate to remove stuck implants of the intramedullary nail or rod from bodies. Mallets are often used to apply an impacting force on a medical tool, such as a chisel, to remove tool from a body. Mallets are also commonly used to insert an implant, and slaphammers to remove stuck implants of the intramedullary nail or rod from bone. While slaphammers are effective, the impacting force must be axially applied to avoid misalignment of the bone, prosthesis, or the inadvertent removal of bone. Moreover, the force applied must be sufficiently accurate to avoid damages.

To overcome some of these problems, slaphammers have been developed and are widely used in orthopaedic procedures to apply an impacting force on various tools used during surgery. However, most slaphammer designs still have several drawbacks. Current slaphammers tend to be very large and heavy, and are thus difficult to handle. Exceptional care must be exercised while using these instruments to prevent injury to the patient and/or the surgeon. In particular, the surgeon's hands can be pinched between the hammer portion of the instrument and the hammer stops. Moreover, the size and weight of the slaphammer can make it very difficult for the surgeon to maintain a steady hand. The size and weight can also result in problems with storage and cleaning.

Accordingly, there remains a need for an automated slaphammer, more compact, lightweight device which can be safely and effectively used to apply extraction force to a medical instrument.

Document EP1834737A1 discloses an electric hand tool device comprising a spindle which can be driven in rotation by a motor and which can be connected to a tool holder at one end, as well as a hammer mechanism for axially moving the spindle.

Document FR2868977A1 discloses a percussion mechanism for a hand-held power tool, in particular for a hammer drill and/or chisel hammer which is provided to transmit impact pulses to a percussion unit which comprises a tool.

### SUMMARY OF THE INVENTION:

The present invention provides a medical instrument to remove stuck implants from bone tool which is useful to accurately and safely apply a force to a medical instrument and, in particular, to a tool used during orthopaedic surgery. The automated slap hammer is designed to provide a safe and accurate procedure for applying a force to a medical device, while minimizing the risk of injury to the patient or to the surgeon's hands during use. The direct force can be directed to remove a medical tool from a location in a patient's body. In addition, the compact design of the instrument provides for ease and accuracy of use, as well as ease of cleaning and storage.

A principal object of the present invention is to provide an automated slap hammer comprising a shaft connected to implants/objects at first end and a second end assembled with, a striking assembly of the shaft rod and movable relative to the striking assembly movement and weight of striking weight. The striking assembly is an assembly of spring member **20,** impactor part A **36,** impactor part B **37,** impacting shaft rod between the first end of the stuck implants rod and the striking member, and impactor part A **36** positioned between the striking weight and the impactor part B **37** is rotated with the help of motor **50** and gear assembly **62** attached to one end of the impactor part B **37**and other end of the impactor part B **37** having groves move the impactor part A **36** having groves and striking assembly **30** apply a proximally directed force and/or a distally directed force on stuck implants.

It is also disclosed a not claimed method for using an automated slaphammer to apply a force to the stuck implants comprises coupling a stuck implants rod to the shaft rod **10,** sliding a striking weight along the face gear **33** to impact on implants in the direction of the shaft rod **10** and against the striking assembly **30,** mechanically retaining the striking weight **31** against the force tension of spring of striking assembly, and mechanically releasing the striking weight to cause the striking weight to slide along the face gear in a direction away from the stuck implants and to deliver an impact to the striking member.

Another object of the present disclosure is to provide, using an automated slaphammer comprises of the impactor part A **36** and impactor part B **37** is positioned adjacent to, or along the side of each other. The impactor part A **36** moves between the first end and second end of the face gear and impact away from the stuck implants. The impactor part A **36** and shaft road are connected with striking spring. The impactor part B **37** is rotated by motor **50** and impactor part A **36**is slidably movable between the face gear **33** first end and second end of the face gear slot.

Apparatus which constitute part of the invention, will become more apparent upon reading the following detailed description of the exemplary embodiments and viewing the drawings.

Another object of the present invention provides an improved orthopaedic device to remove stuck implants such as intramedullary nail or rod and the instruments associated therewith.

An object of the present invention is an automated system that helps surgeon to deliver controlled force to implant in order to remove it from bone safely.

It also provides an orthopaedic device wherein the grooves in the device controls the force exerted to remove stuck implants of the intramedullary nail or rod.

It also provides an improved orthopaedic device with a motor attachment to control revolution per minute of the device.

It also provides an improved orthopaedic device to control the striking force by adjusting spring force.

### BRIEF DESCRIPTION OF THE DRAWINGS:

The drawings constitute a part of this invention and include exemplary embodiments of the present invention illustrating various objects and features thereof.
**Figure 1****.**is the schematic view of slap hammer
**Figure 2****.**is the external view of slaphammer
**Figure 3** is the cross-sectional view of the slaphammer
**Figure 4** is the horizontal view of the slaphammer
**Figure 5** is the cross sectional view of grove assembly
**Figure 6** is the exploded view of an automated slaphammer withdrawal or removal tool mated to a medical instrument on stuck implants/objects.

### DETAILED DESCRIPTION OF THE INVENTION:

Reference will now be made in detail to exemplary embodiments of the invention as illustrated in the accompanying drawings, in which like reference characters designate like or corresponding parts throughout the drawings. It should be noted, however, that the invention in its broader aspects is not limited to the specific details, representative devices and illustrative examples shown and described in this section in connection with the exemplary embodiments. The invention according to its various aspects is particularly pointed out and distinctly claimed in the attached claims read in view of this specification. The present invention provides a medical instrument automated slaphammer which is useful to accurately and safely apply a force to a medical instrument and, in particular, to a tool used during withdrawal or removal of stuck implants/objects form Bone during orthopaedic surgery. The instrument is designed to provide a safe and accurate procedure for applying a force to a medical device, while minimizing the risk of injury to the patient or to the surgeon's hands during use. The withdrawal or removal force can be directed to withdraw a stem/implant which was implanted during previous orthopaedic surgery to support bone or reconstruct normal skeletal structure (i.e broken bone, damaged joints) from a location in a patient's body, to prepare an implant site using a broaching device or similar bone preparation device.

The present invention provides an automated system that helps the surgeon to deliver controlled extraction force to implant in order to remove it from bone and femoral stems in revision hip surgery.

The present invention provides an automated tool used to apply force towards or away from a surgical area in a joint replacement procedure is known as "automated slap hammer" or "automated slide hammer". Slap hammers, also known by the alternate name of a "slide hammer", provide a weight capable of sliding about a guide rod or face gear. The sliding weight can be used to generate a force when the sliding weight reaches and strikes a stop provided on the guide rod or the face gear. For example, a slap hammer tool can be used to provide force downward towards an object, to provide precision force (e.g., impact) towards a point of interest at an end of the slap hammer. Likewise, a slap hammer tool can be used to provide force away from a stuck implants rod, such as to extract ans tuck implants rod coupled to an shaft rod 10 end of the slap hammer tool.

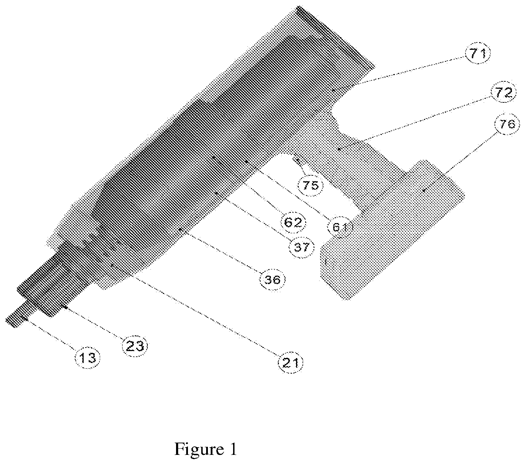

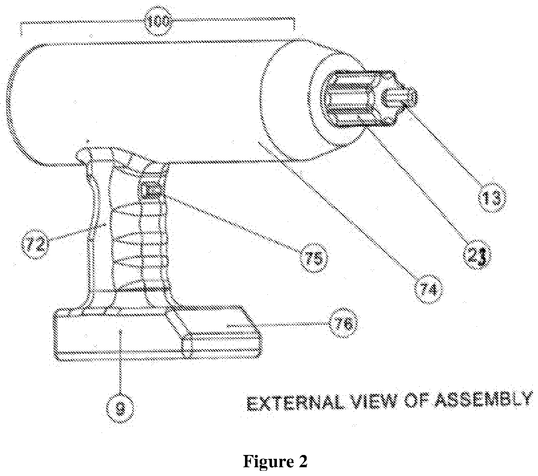

Figure 1 and 2 is an illustration of a slap hammer device **100** according to an embodiment of the invention. The slap hammer **100** includes a screw coupling mechanism **13** which will be coupled to the surgical implant **70**(not shown). The coupling mechanism **13** is joined to the knob **23** which is internally connected to spring member/ spring tensioner/spring groove member **20** which is used to adjust the tension of the spring element **21** of the slaphammer, by rotating tension member through **23.** The body of the slaphammer **74** consists of the striking assembly **30,** grove assembly **38** and the gear assembly **62.** The handle of the slaphammer **72** consists of a stand **9,** button **75** and battery pack **76** which also act as counter balance while operating instrument. The slaphammer can be operated electrically, manually or by solar energy. The striking assembly **30** consists of striking weight **31,** impactor part A **36** and impactor part B **37.** The striking assembly **30** is connected to the gear assembly **62** with an intermediate disc **61** which is attached to the motor **50.**

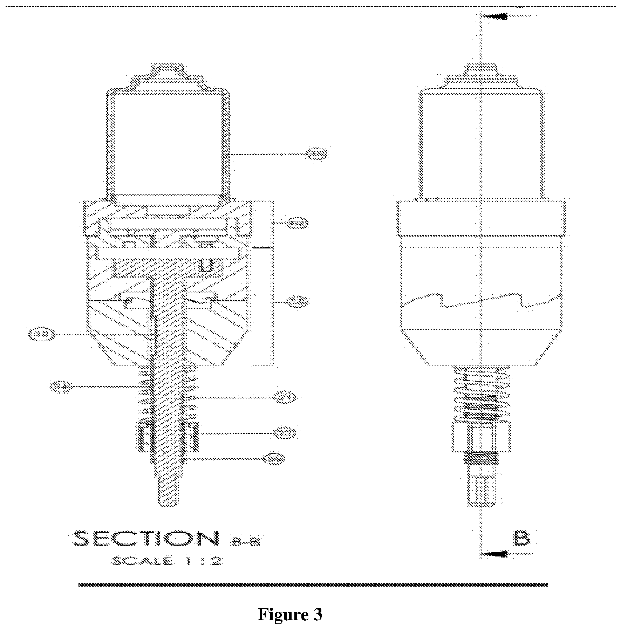

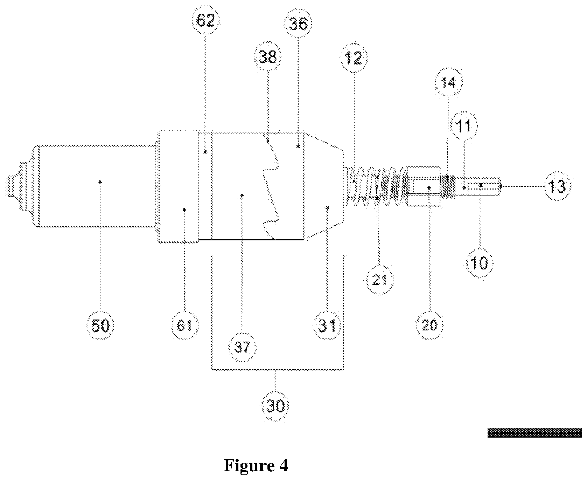

Figure 3 and Figure 4 illustrates cross sectional and horizontal view of slap hammer. The slaphammer **100** generally includes a shaft rod **10** having a threaded portion **14** arranged on a distal end **11** and a spring element **21** arranged on a proximal end **12** with striking assembly **30.** The spring member/spring tensioner/spring groove member **20** will engage with the knob **23** to adjust the tension of the spring element **21.The** spring member, spring tensioner or spring groove member can be used in the embodiment of the invention. The striking assembly **30** consists of striking weight **31,** impactor part A **36** and impactor part B **37.** The striking assembly **30** is connected to the gear assembly **62** which is attached to the motor **50.** When motor drives impactor part B **37** in rotational movement, impactor part A **36** moves axially because of key and slot **32** mentioned in FIG. 3.Thus the key slot maintains the axial movement of the rod. The zig zag groove assembly **38** also maintains the movement of the shaft rod **10.** A spring member **20,** illustrated in FIG. 3 as a spring element **21,** is positioned about the face gear **33** at the second end **35** of face gears **33.** The spring element **21** comprises a cylindrical compression spring designed to resist applied compression force and to store energy in a compressed stance. The spring member **20** may comprise other mechanisms, such as a tensioning spring. As the striking weight **31** is moved toward the second end **35** of the face gear **33,** the spring element **21** is compressed to store energy and striking weight **31** in the opposite direction, towards the first end **34** of face gears **33.**

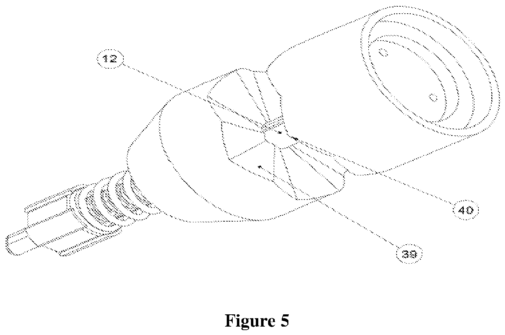

Figure 5 illustrates cross sectional view of the grooves of the of slap hammer, which is a zig-zag assembly maintaining the axial movement of the shaft rod. When an operator wants to activate the slap hammer, the triggering mechanism can be triggered by rotating impactor part B **37** and displacing the groove B **40** over groove A **39** sliding impactor part A **36** and the along the guide rod **33.** As the triggering mechanism moves the striking assembly **30** and impactor part A **36** impacts the shaft rod **10** at the distal end **11** attached to the stuck implants rod. As used herein "a groove" is a long and narrow indentation built into a material, generally for the purpose of allowing another material or part to move within the groove and be guided by it. It is however to be noted that the shape of the groove does not limit the scope of invention.

Devices according to embodiments of the present invention may be fabricated from light, strong and rigid biocompatible materials. For example, in some embodiments, the slap hammer may comprise metal, metal alloys, polymeric composites or other known suitable materials. In some embodiments, various components or the entire slap hammer may be disassembled or taken apart for storage and/or cleaning. In some embodiments, biasing elements of differing strengths, or tensions, may be utilized, to provide varying forces on objects coupled to the slap hammer.

Various slap hammers may be configured in many different dimensions, and deliver a wide range of impact forces. In some embodiments, the slap hammer may be dimensioned for delivering substantially large impact forces and in other embodiments, the slap hammer may be dimensioned for delivering lesser impact forces. Advantageously, the adjustable striking assembly **30** allows slap hammers **100** of any dimension to deliver a wide range of impact forces.

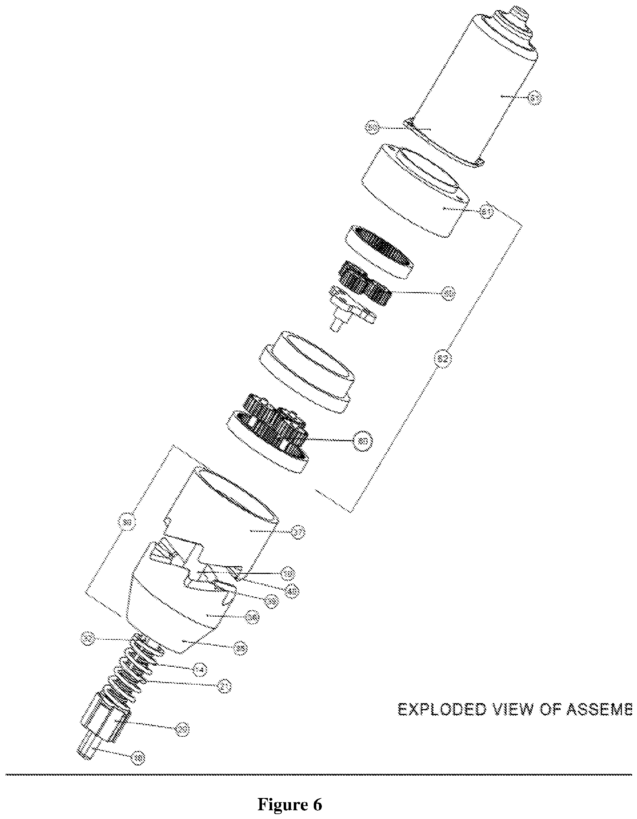

With initial reference to Figure 6, an exploded view of slap hammer for in-vivo assembly of implants according to the present teachings is shown and generally identified at reference. The slaphammer **100** generally includes a shaft rod **10** having a threaded portion **14** arranged on a distal end **11** and a spring member **20** arranged on a proximal end **12** with striking assembly **30.** The slap hammer **100** also includes striking weight **31** that is slidable along zig zag face gears groove assembly **38** and spring member **14.** The slap hammer **100** is used to apply a force to stuck implants rod coupled to the shaft rod **10.** Shaft **10** consists of a key slot with key **32,** which restricts rotation of Impactor A **36** but allows the axial movement.

The shaft rod **10** can be coupled to an object, such as a surgical implant, prosthesis, or IMN, in a variety of ways using coupling mechanism **13.** As shown in FIG. 6, the threaded portion **14** of the shaft rod **10** comprises a screw coupling mechanism **13.** The coupling mechanism **13** comprises a threaded end configured to mate with a threaded portion of a surgical implement. Other coupling mechanisms include pliers, a hook, a clamp, a ring, a magnet, and like coupling mechanisms. Still other coupling mechanisms may be used, such as a snap fit or friction fit connection. In some scenarios, a removable or interchangeable coupling mechanism may be desirable, so that the slap hammer may be quickly disconnected from one surgical implement and connected to another. In other embodiments a more permanent coupling is used to provide greater stability and rigidity.

As shown in Figure. 6, the striking weight **31** moves, or slides, along zig zag groove of the face gears **38** via a key slot **32** in the striking weight **31.** Face gears **38** extends through the key slot **32,** and the striking weight can move toward spring member **20** and away from the first end **34** of face gears **38.**

A spring member **20,** illustrated in FIG. 6 as a spring element **21,** is positioned about the face gear **33** at the second end **35** of face gears groove assembly **38.** The spring element **21** comprises an axial compression spring designed to resist applied compression force and to store energy in a compressed stance. The spring member **20** may comprise other mechanisms, such as a tensioning spring. As the striking weight **31** is moved toward the second end 35 of the rod **33,** the spring element **21** is compressed to store energy and striking weight **31** in the opposite direction, towards the first end **34** of face gears **38.**

A releasable retaining mechanism, retains the striking weight **31** against the action of the spring element **21.** A groove mechanism **38** for mechanically triggering the release of impactor part A **36** and the striking weight **31** is coupled to the impactor part B **37.** The groove mechanism **38** consist of grooves A**39**on impactor part A **36** and grooves B **40** on impactor part B **37** mechanically trigger, releasing the striking weight **31** which is propelled by the spring element **21** in the opposite direction, towards the first end **34** of the face gear **33** towards the impactor part B **37.**

The striking weight **31** is adapted to rotate along face gears **38** between the spring element **21** and impactor part B **37** with releasable retaining mechanism and slides the striking weight **31** between the first ends 34 at the second end **35** of face gears 38. The spring element **14** is disposed between the striking weight **31** and the shaft rod **11**proximal end **12,** the spring element **21** releases and urges the striking weight **31** in the direction of the impactor part B **37.** The striking weight **31** may be urged against the action of the spring **21** and locked in place via the releasable retaining mechanism with impactor part B **37.**

When an operator wants to activate the slap hammer, the triggering mechanism can be triggered by rotating impactor part B **37** and displacing the groove B **40** over groove

A **39** sliding impactor part A **36** and the along theface gears **33.** As the triggering mechanism moves the striking assembly **30** and impactor part A **36** impacts the shaft rod **10** at the distal end **11** attached to the stuck implants rod. The striking weight **31** of impactor part A **36** assembled moves towards the spring member **20** at proximal end **12** of the shaft rod **11.** The spring member **20** store potential energy in the form of a compression spring will compress and store potential energy. The spring member **20** is attached to the spring knob **23** and spring element **21** which maintains the spring member **20.** The striking weight **31** of impactor part A **36** is released in the direction towards impactor part B **37** with great impact on shaft rod **11** at the distal end **18** attached to the stuck implants rod. The impactor part B **37** is rotated with the help of motor **50** and gear assembly **62** attached to one end of the motor **50** and other end of the impactor part B **37** having grooves B move the impactor part A **36** having grooves A and striking assembly apply a proximally directed force and/or push the striking weight **31** upward a distally directed force on stuck implants towards the spring member **20** which adjusts the compression of the spring element **21.**

Figure 6 which is is a exploded view of a slap hammer according to another embodiment of the invention. In figure 6, a slap hammer comprises a zig zag face 38 having a first end **34** and a second end **35.** The slap hammer also includes a striking weight **31** that slides along the face gear **33.** Face gears **38** of the slap hammer shown in further comprises a coupling mechanism in the form of coupling mechanism **13.** The coupling mechanism **13** can be screwed, clamped, or hooked clamp on an object to be removed from a surgical area. Once the coupling mechanism **13** are screwed, clamped, or hooked to an object, the striking weight **31** can be released at the first end **34** of the face gear, and propelled by the tension of the spring element **21** towards the second end **35** of face gears **38,** where it will impact the striking assembly **30,** and impart a pulling force on the object coupled by the coupling mechanism **13** with shaft rod **10.**

Figure 6 is an illustration of various connecting mechanisms according to embodiments of the invention. The first end **34** of the shaft rod **10** can be configured to accept a variety of different connecting mechanisms, such as screw, clamped or pliers. Other off-the-shelf and/or custom made products can be used. As shown in figure 6, other connecting mechanisms comprise various threaded screw endings. Alternatively, various connecting mechanisms comprise pin connectors which are advantageous for clamping around smaller objects, such as wire.

Figure 6 is an illustration of a using a slap hammer according to another embodiment of the invention. The method of coupling mechanism **13** by coupling a shaft rod to an object. The shaft rod **10** may be coupled to the object, i.e. the target workpiece, via a coupling mechanism, such as a threaded screw, pliers, a clamp, a ring, or a hook. In other embodiments, other coupling mechanisms may be used, such as a magnet, or a vacuum suction coupling mechanism. The object coupled to the guide rod may be surgical instrument, or some other object.

The force applied to the object may be controlled by optionally adjusting the position of a spring member **20** on the shaft rod **10** by rotating knob **23.** The striking assembly **30** is positioned on a second end of the shaft rod **10** with spring member **20** and the object coupled to the shaft rod **10** (i.e. the target workpiece) on the first end of the shaft rod **10.** By moving the impactor part B **37** the striking assembly **30** relative to the first end of the face gear, the acceleration of the striking weight **31** may be increased or decreased by adjusting the spring member **20** by controlling the spring tension force, and thus the force delivered by the striking weight **31** may be adjusted. The striking assembly **30** can be adapted to move along the gears toward or away from the first end of the face gear **33** in order to adjust the position of the striking assembly **24** relative to the striking weight.

After the shaft rod **10** is coupled to the object **70** and the acceleration of the striking weight **31** is adjusted, the impactor part B **37** is rotated, striking assembly **30** slid, along the face gear 33 towards the object and against the spring member **20.** As the sliding weight is slide towards the object **70,** the spring member **20** store potential energy. For example, spring member **20** in the form of a compression spring will compress and store potential energy.

While or immediately after the striking weight **31** is positioned against the spring member **20,** the striking weight **31** is mechanically retained against the spring member **20.** A temporary locking mechanism, i.e. a releasable retaining mechanism, releasable retains the striking weight **31** against the spring member **20.**

Finally, the striking weight **31** is mechanically released from the temporary locking mechanism, causing the striking weight **31** to slide away from the object along the face gear **33** to deliver an impact to the shaft rod **10.** A triggering mechanism may mechanically release the striking weight **31** from the temporary locking mechanism. When the striking weight **31** impacts the shaft rod **10,** the slap hammer moves or jerks in the same direction traveled by the striking weight, potentially moving or dislodging the object coupled to the slap hammer in the same direction.

As it is described in the diagram below, implant is attached to shaft of the device with specific attachment. The surgeon now applies traction to handle, and starts machine by using regulator.

The regulator button **75** allows the surgeon to run machine at specific frequency of reverse impacts. The present invention is an automated system wherein the surgeon can apply more force to implant compared to hammer, face gears and rod.

The presently disclosed slap hammer provides numerous advantages in the art. For example, the disclosed slap hammer allows a user to have greater control over the force of the hammer strike than in conventional slap hammers. Specifically, disclosed slap hammers can deliver variable forces, allowing a single device to deliver a range of controlled impact forces.

This invention is susceptible to considerable variation in its practice. Therefore the foregoing description is not intended to limit, and should not be construed as limiting the invention to the particular exemplifications presented hereinabove. Rather, what is intended to be covered is as set forth in the ensuing claims.

## Claims

1. An automated slap hammer for removing stuck implants, the automated slap hammer comprising:
a) a shaft rod (10) configured to be connected to stuck implants rod at a first end of the shaft rod (10);
b) a face gear (33);
c) a spring member (20) positioned about the face gear (33);
d) a striking assembly (30) assembled to a second end of the shaft rod (10) with the spring member (20), the striking assembly (30) comprising an impactor part A (36), an impactor part B (37) and a striking weight (31), the impactor part A (36) being positioned between the striking weight (31) and the impactor part B (37), the striking assembly (30) and the impactor part A (36) being configured for impacting the shaft rod (10) at a distal end (11) attached to the stuck implants rod;
e) a motor (50) and a gear assembly (62), the gear assembly (62) being attached to one end of the motor (50) and to other end of the impactor part B (37), the impactor part B (37) being configured to be rotated by the motor (50) and gear assembly (62) so that the striking assembly (30) apply a proximally directed force to a distally directed force on stuck implants; and
f) a groove mechanism (38) comprising grooves A (39) on the impactor part A (36) and grooves B (40) on the impactor part B (37), the groove mechanism (38) being configured so that by rotating the impactor part B (37) and displacing the grooves B (40) over groves A (39) the striking assembly (30) slides along the face gear (33).

2. An automated slap hammer according to claim 1 further comprising a screw coupling mechanism (13) at a first end (34) of a face gear (33) coupled to the spring member (20).

3. An automated slap hammer according to claim 1, wherein the spring member (20) comprises a spring element (21) and a knob (23).

4. An automated slap hammer according to claim 1, wherein the shaft rod (10) comprises a threaded portion 14 that comprises a screw coupling mechanism (13).

5. An automated slap hammer according to claim 4, wherein the coupling mechanism (13) comprises a threaded end configured to mate with a threaded portion of a surgical implant.

6. An automated slap hammer according to claim 5, wherein the shaft rod (10) is configured to be coupled to an object, such as a surgical implant, prosthesis, or IMN, in a variety of ways using coupling mechanism (13).

7. An automated slap hammer according to claim 1, further comprising a button that is used to start, stop, and control striking frequency of slap hammer.

8. An automated slap hammer according to claim 1, wherein the groove mechanism (38) is configured to maintain the axial movement of impactor A (36).

9. An automated slap hammer according to claim 1, wherein the groove mechanism (38) consists of grooves in a device that controls the force exerted to remove stuck implants of an intramedullary nail or rod.

10. An automated slap hammer according to claim 1 wherein, the shaft rod (10) consists of a key slot with key (32), which restricts rotation of the impactor A (36) but allows the axial movement.

11. An automated slap hammer according to claim 1, wherein the striking member consists of a striking weight (31) configured to guide the face gear (33) to impact in operative manner on stuck implants in the direction of the shaft rod (10) and against the striking assembly (30), being further configured to mechanically retain the striking weight (31) against the force tension of spring member (20) of the striking assembly (30), and being further configured to mechanically release the striking weight (31) to cause the striking weight (31) to slide along the face gear (33) in a direction away from the stuck implants and, is further configured to deliver an impact to the striking member when is in operative manner.

12. An automated slap hammer according to claim 1, wherein it further comprises:
a. means for coupling a stuck implants rod to the shaft rod (10);
b. means for sliding a striking weight (31) along the face gear (33) to impact on stuck implants in the direction of the shaft rod (10) and against the striking assembly (30);
c. means for mechanically retaining the striking weight (31) against the force tension of spring member (20) of the striking assembly (30) and;
d. means for mechanically releasing the striking weight (31) to cause the striking weight (31) to slide along the face gear (33) in a direction away from the stuck implants and to deliver an impact to the shaft rod (10).

13. An automated slap hammer according to claim 1, wherein:
a. the impactor part A (36) and impactor part B (37) are positioned adjacent along the side of each other;
b. the impactor part A (36) is configured to move between a first end (34) and second end (35) of the face gear (33) and to impact away from the stuck implants;
c. the impactor part A (36) and shaft rod (10) are connected with the spring member (20);
d. the impactor part B (37) is configured to be rotated by the motor (50) and the gear assembly (62), and the impactor part A (36) is configured to be axially movable between the face gear (33) first end (34) and second end (35) of the face gear (33) slot.

## Patentansprüche

1. Automatisierter Schlaghammer zum Entfernen gesteckter Implantate, wobei der automatisierte Schlaghammer Folgendes umfasst:
a) einen Schaftstab (10), der dazu ausgebildet ist, an einem ersten Ende des Schaftstabs (10) mit gesteckten Implantatstäben verbunden zu werden;
b) ein Planrad (33);
c) ein Federteil (20), das um das Planrad (33) herum positioniert ist;
d) eine Schlagbaugruppe (30), die an einem zweiten Ende des Schaftstabs (10) mit dem Federteil (20) montiert ist, wobei die Schlagbaugruppe (30) ein Schlagkörperteil A (36), ein Schlagkörperteil B (37) und ein Schlaggewicht (31) umfasst, wobei das Schlagkörperteil A (36) zwischen dem Schlaggewicht (31) und dem Schlagkörperteil B (37) positioniert ist, wobei die Schlagbaugruppe (30) und das Schlagkörperteil A (36) dazu ausgebildet sind, an einem distalen Ende (11) auf den an dem gesteckten Implantatstab befestigten Schaftstab (10) zu schlagen;
e) einen Motor (50) und eine Getriebebaugruppe (62), wobei die Getriebebaugruppe (62) an einem Ende des Motors (50) und an dem anderen Ende des Schlagkörperteils B (37) befestigt ist, wobei das Schlagkörperteil B (37) dazu ausgebildet ist, von dem Motor (50) und der Getriebebaugruppe (62) in Drehung versetzt zu werden, sodass die Schlagbaugruppe (30) eine proximal gerichtete Kraft auf eine distal gerichtete Kraft auf gesteckten Implantaten aufbringt; und
f) einen Nutenmechanismus (38) mit Nuten A (39) an dem Schlagkörperteil A (36) und Nuten B (40) an dem Schlagkörperteil B (37), wobei der Nutenmechanismus (38) so ausgebildet ist, dass durch Drehen des Schlagkörperteils B (37) und Verschieben der Nuten B (40) über die Nuten A (39) die Schlagbaugruppe (30) an dem Kronenrad (33) entlanggleitet.

2. Automatisierter Schlaghammer nach Anspruch 1, der ferner einen Schraubkopplungsmechanismus (13) an einem ersten Ende (34) eines mit dem Federteil (20) verbundenen Planrads (33) umfasst.

3. Automatisierter Schlaghammer nach Anspruch 1, wobei das Federteil (20) ein Federelement (21) und einen Knauf (23) umfasst.

4. Automatisierter Schlaghammer nach Anspruch 1, wobei der Schaftstab (10) einen Gewindeabschnitt (14) umfasst, der einen Schraubkopplungsmechanismus (13) umfasst.

5. Automatisierter Schlaghammer nach Anspruch 4, wobei der Kopplungsmechanismus (13) ein mit einem Gewinde versehenes Ende umfasst, das dazu ausgebildet ist, in einen Gewindeabschnitt eines chirurgischen Implantats einzugreifen.

6. Automatisierter Schlaghammer nach Anspruch 5, wobei der Schaftstab (10) dazu ausgebildet ist, mit einem Gegenstand wie zum Beispiel einem chirurgischen Implantat, einer Prothese oder IMN, auf viele verschiedene Arten unter Verwendung eines Kopplungsmechanismus (13) verbunden zu werden.

7. Automatisierter Schlaghammer nach Anspruch 1, der ferner einen Knopf umfasst, der dazu verwendet wird, einen Schlaghammer zu starten, zu stoppen und seine Schlagfrequenz zu steuern.

8. Automatisierter Schlaghammer nach Anspruch 1, wobei der Nutenmechanismus (38) dazu ausgebildet ist, die Axialbewegung des Schlagkörpers A (36) beizubehalten.

9. Automatisierter Schlaghammer nach Anspruch 1, wobei der Nutenmechanismus (38) aus Nuten in einer Vorrichtung besteht, die die zum Entfernen von aus einem intramedullären Nagel oder Stab bestehenden gesteckten Implantaten ausgeübte Kraft steuert.

10. Automatisierter Schlaghammer nach Anspruch 1, wobei der Schaftstab (10) aus einer Keilnut mit einem Keil (32) besteht, der die Drehung des Schlagkörpers A (36) einschränkt, jedoch die Axialbewegung erlaubt.

11. Automatisierter Schlaghammer nach Anspruch 1, wobei das Schlagelement aus einem Schlaggewicht (31) besteht, das dazu ausgebildet ist, das Planrad (33) zu führen, um in operativer Weise auf gesteckte Implantate in Richtung des Schaftstabs (10) und gegen die Schlagbaugruppe (30) zu schlagen, und ferner dazu ausgebildet ist, das Schlaggewicht (31) mechanisch gegen die Spannkraft des Federteils (20) der Schlagbaugruppe (30) festzuhalten, und ferner dazu ausgebildet ist, das Schlaggewicht (31) mechanisch zu lösen, damit das Schlaggewicht (31) in eine Richtung weg von den gesteckten Implantaten an dem Planrad (33) entlanggleiten kann, und ferner dazu ausgebildet ist, einen Schlag auf das Schlagelement auszuüben, wenn dieses operativ ist.

12. Automatisierter Schlaghammer nach Anspruch 1, wobei er ferner Folgendes umfasst:
a. Mittel zum Verbinden eines gesteckten Implantatstabs mit dem Schaftstab (10);
b. Mittel zum Schieben eines Schlaggewichts (31) an dem Planrad (33) entlang, um auf gesteckte Implantate in Richtung des Schaftstabs (10) und gegen die Schlagbaugruppe (30) zu schlagen;
c. Mittel zum mechanischen Festhalten des Schlaggewichts (31) gegen die Spannkraft des Federteils (20) der Schlagbaugruppe (30); und
d. Mittel zum mechanischen Lösen des Schlaggewichts (31), damit das Schlaggewicht (31) in eine Richtung weg von den gesteckten Implantaten an dem Planrad (33) entlanggleiten kann und einen Schlag auf den Schaftstab (10) ausüben kann.

13. Automatisierter Schlaghammer nach Anspruch 1, wobei:
a. das Schlagkörperteil A (36) und das Schlagkörperteil B (37) seitlich aneinander angrenzend positioniert sind;
b. das Schlagkörperteil A (36) dazu ausgebildet ist, sich zwischen einem ersten Ende (34) und einem zweiten Ende (35) des Planrads (33) zu bewegen und von den gesteckten Implantaten weg zu schlagen;
c. das Schlagkörperteil A (36) und der Schaftstab (10) mit dem Federteil (20) verbunden sind;
d. das Schlagkörperteil B (37) dazu ausgebildet ist, von dem Motor (50) und der Getriebebaugruppe (62) in Drehung versetzt zu werden, und das Schlagkörperteil A (36) dazu ausgebildet ist, zwischen dem ersten Ende (34) des Planrads (33) und dem zweiten Ende (35) des Planrads (33) axial bewegbar zu sein.

## Revendications

1. Un marteau à frapper automatisé pour retirer des implants coincés, le marteau à frapper automatisé comprenant :
a) une tige (10) formant arbre configurée pour être connectée, au niveau d'une première extrémité de la tige (10) formant arbre, à une tige d'implants coincés ;
b) un équipement facial (33) ;
c) un élément ressort (20) positionné autour de l'équipement facial (33) ;
d) un ensemble de frappe (30) assemblé à une deuxième extrémité de la tige (10) formant arbre avec l'élément ressort (20), l'ensemble de frappe (30) comprenant une partie d'impacteur A (36), une partie d'impacteur B (37) et un poids de frappe (31), la partie d'impacteur A (36) étant positionnée entre le poids de frappe (31) et la partie d'impacteur B (37), l'ensemble de frappe (30) et la partie d'impacteur A (36) étant configurés pour impacter la tige (10) formant arbre au niveau d'une extrémité distale (11) fixée à la tige d'implants coincés ;
e) un moteur (50) et un ensemble d'engrenage (62), l'ensemble d'engrenage (62) étant fixé à une extrémité du moteur (50) et à l'autre extrémité de la partie d'impacteur B (37), la partie d'impacteur B (37) étant configuré pour être mis en rotation par le moteur (50) et l'ensemble d'engrenage (62) de sorte que l'ensemble de frappe (30) applique une force dirigée de manière proximale à une force dirigée de manière distale sur des implants coincés ; et
f) un mécanisme à rainures (38) comprenant des rainures A (39) sur la partie d'impacteur A (36) et des rainures B (40) sur la partie d'impacteur B (37), le mécanisme à rainures (38) étant configuré de sorte qu'en faisant tourner la partie d'impacteur B (37) et en déplaçant les rainures B (40) sur les rainures A (39), l'ensemble de frappe (30) coulisse le long de l'équipement facial (33).

2. Un marteau de frappe automatisé selon la revendication 1, comprenant en outre un mécanisme (13) de liaison de vis à une première extrémité (34) d'un équipement facial (33) relié à l'élément ressort (20).

3. Un marteau de frappe automatisé selon la revendication 1, dans lequel l'élément ressort (20) comprend un élément (21) formant ressort et un bouton (23).

4. Un marteau à frapper automatisé selon la revendication 1, dans lequel la tige (10) formant arbre comprend une partie filetée 14 qui comprend un mécanisme (13) de liaison de vis.

5. Un marteau à frapper automatisé selon la revendication 4, dans lequel le mécanisme de liaison (13) comprend une extrémité filetée configurée pour coopérer avec une partie filetée d'un implant chirurgical.

6. Un marteau à frapper automatisé selon la revendication 5, dans lequel la tige (10) formant arbre est configurée pour être reliée à un objet, tel qu'un implant chirurgical, une prothèse ou un IMN, de diverses manières en utilisant un mécanisme de liaison (13).

7. Un marteau à frapper automatisé selon la revendication 1, comprenant en outre un bouton qui est utilisé pour démarrer, arrêter et commander la fréquence de frappe du marteau à frapper.

8. Un marteau de frappe automatisé selon la revendication 1, dans lequel le mécanisme à rainure (38) est configuré pour maintenir le mouvement axial de l'impacteur A (36).

9. Un marteau à frapper automatisé selon la revendication 1, dans lequel le mécanisme à rainures (38) consiste en des rainures dans un dispositif qui commande la force exercée pour retirer des implants coincés d'un clou ou d'une tige intramédullaire.

10. Un marteau de frappe automatisé selon la revendication 1, dans lequel la tige (10) formant arbre est constituée d'une rainure de clavette avec clavette (32), qui limite la rotation de l'impacteur A (36) mais autorise le mouvement axial.

11. Un marteau à frapper automatisé selon la revendication 1, dans lequel l'élément de frappe consiste en un poids de frappe (31) configuré pour guider l'équipement facial (33) pour frapper de manière opérationnelle sur des implants coincés dans la direction de la tige (10) formant arbre, et contre l'ensemble de frappe (30), étant en outre configuré pour retenir mécaniquement le poids de frappe (31) contre la force de tension de l'élément ressort (20) de l'ensemble de frappe (30), et étant en outre configuré pour libérer mécaniquement le poids de frappe (31) pour amener le poids de frappe (31) à coulisser le long de l'équipement facial (33) dans une direction allant en s'éloignant des implants coincés et, est en outre configuré pour produire un impact sur l'élément de frappe lorsqu'il est opérationnel.

12. Un marteau de frappe automatisé selon la revendication 1, qui comprend en outre :
a. des moyens pour relier une tige d'implants coincés à la tige (10) formant arbre ;
b. des moyens pour faire coulisser un poids de frappe (31) le long de l'équipement facial (33) pour produire des impacts sur des implants coincés dans la direction de la tige (10) formant arbre et contre l'ensemble de frappe (30) ;
c. des moyens pour retenir mécaniquement le poids de frappe (31) contre la force de tension de l'élément ressort (20) de l'ensemble de frappe (30) et ;
d. des moyens pour libérer mécaniquement le poids de frappe (31) de façon à amener le poids de frappe (31) à coulisser le long de l'équipement facial (33) dans une direction allant en s'éloignant des implants coincés et à produire un impact sur la tige (10) formant arbre.

13. Un marteau de frappe automatisé selon la revendication 1, dans lequel :
a. la partie d'impacteur A (36) et la partie d'impacteur B (37) sont positionnées de façon adjacente l'une à côté de l'autre ;
b. la partie d'impacteur A (36) est configurée pour se déplacer entre une première extrémité (34) et une deuxième extrémité (35) de l'équipement facial (33) et pour produire des impacts s'éloignant des implants coincés ;
c. la partie d'impacteur A (36) et la tige (10) formant arbre sont reliées à l'élément ressort (20) ;
d. la partie d'impacteur B (37) est configurée pour être entraînée en rotation par le moteur (50) et l'ensemble d'engrenage (62), et la partie d'impacteur A (36) est configurée pour être mobile axialement entre la première extrémité (34) et la deuxième extrémité (35) de l'équipement facial (33) de la fente de l'équipement facial (33).
